# EUROPÄISCHE PATENTSCHRIFT

(11) **EP 1 434 537 B1**
(45) Veröffentlichungstag und Bekanntmachung des Hinweises auf die Patenterteilung: **22.06.2005**
(21) Anmeldenummer: 03770972.2
(22) Anmeldetag: 11.10.2003
(51) Int. Cl.: A61F 2/04, A61F 2/06

(54) **Vorrichtung zum Komprimieren rohrförmiger Endoprothesen, sowie zum Einführen einer komprimierten Endoprothese in ein Applikationsrohr**
Device for compressing tubular endoprostheses and for inserting a compressed endoprosthesis in an application tube
Dispositif pour comprimer des endoprothèses tubulaires et pour introduire une endoprothèse comprimée dans un tube d' application

(30) Priorität: 26.10.2002 DE 10249927
(43) Veröffentlichungstag der Anmeldung: 07.07.2004
(73) Patentinhaber: Karl Storz GmbH & Co. KG, 78532 Tuttlingen (DE)
(72) Erfinder: Eisenkolb, Peter, 87435 Kempten (DE)
(74) Vertreter: Hofmeister, Frank Horst
(86) Internationale Anmeldenummer: PCT/EP2003/011281
(87) Internationale Veröffentlichungsnummer: WO 2004/037124

(56) Entgegenhaltungen:
- EP-A- 0 519 282
- DE-A- 10 004 979
- US-A- 5 676 671
- US-A1- 2002 040 236

## Beschreibung

Die Erfindung betrifft eine Vorrichtung zum Komprimieren rohrförmiger Endoprothesen, insbesondere Stents, sowie zum Einführen einer komprimierten Endoprothese in ein Applikationsrohr.

Unter Endoprothesen versteht man in der Chirurgie endoskopisch plazierte Röhrchen, sogenannte Stents oder Tubusse, zur Überbrückung oder Drainage bei Stenosen, Strikturen und Tumoren. Bei den Stents handelt es sich um aus verschiedenen körperverträglichen Materialien herstellbaren selbstexpandierenden rohrförmigen Prothesen, die sich nach der endoskopischen oder radiologischen Implantation selbsttätig ausdehnen, um beispielsweise bei tumorbedingter Stenose und Obstruktion oder bei arteriosklerotisch bedingter kurzstreckiger Gefäßstenose, das Lumen eines Hohlorgans zu überbrücken oder zu erhalten.

Die Implantation der Endoprothesen erfolgt in der Regel über ein Applikationsrohr, in das die Prothese in einem komprimierten bzw. gefalteten Zustand eingesetzt wird, um im Operationsgebiet unter endoskopischer Beobachtung über ein geeignetes Ausdrückwerkzeug wieder aus dem Applikationsrohr herausgedrückt zu werden. Im Operationsgebiet entfaltet sich die Endoprothese dann wieder, um sich an der vorgesehenen Stelle des Hohlorgans festlegen zu können.

Aus der DE 100 04 979 A1 ist ein System zum Einsetzen von Endoprothesen bekannt, das neben der Vorrichtung zum Komprimieren rohrförmiger Endoprothesen sowie zum Einführen einer komprimierten Endoprothese in ein Applikationsrohr auch das vollständige endoskopische Operationsset umfasst, um die Prothese über das Applikationsrohr in das Operationsgebiet einzubringen. Bei diesem bekannten System erfolgt das Komprimieren und Einsetzen der Endoprothese in das Applikationsrohr mittels einer aus zwei aufeinander schiebbarer Rohrstücken bestehenden Komprimier- und Einführvorrichtung, die ihrerseits an dem Applikationsrohr festlegbar ist.

Um die Endoprothese vollständig komprimiert, das heißt ein Längswulst bildend eingefaltet in das Applikationsrohr einzufügen, wird die Prothese zunächst manuell der Länge nach so eingedrückt, dass sich eine nach innen in den hohlen Innenraum der Prothese erstreckende Längsfalte bildet. Diese solchermaßen manuell vorkomprimierte Endoprothese wird jetzt manuell in das äußere Rohrstück der Komprimier- und Einführvorrichtung eingesetzt. Anschließend wird das freie Ende dieses Rohrstückes über einen Stopfen verschlossen und das mit der Prothese versehene Rohrstück in Richtung auf das innere der beiden Rohrstücke der Kornprimier- und Einführvorrichtung verschoben, welches auf dem Applikationsrohr festgelegt ist. Durch dieses Verschieben der Rohrstücke gegeneinander wird die Endoprothese in das Innere des Applikationsrohres gedrückt und dabei weiter komprimiert.

Neben dem Umstand, dass die aus den beiden Rohrstücken bestehende Komprimier- und Einführvorrichtung im Aufbau und in der Handhabung sehr aufwendig und komplex ist, ist bei diesem bekannten System nachteilig, dass die Endoprothese zunächst manuell gefaltet und in die Komprimier- und Einführvorrichtung eingeführt werden muß. Da die Endoprothesen in der Regel mit einem Gleitgel benetzt sind, lassen sich die Prothesen schlecht manuell handhaben. Darüber hinaus geht Gleitgel verloren und werden die Hände des Operateurs mit Gleitgel benetzt, was für die weitere Arbeit hinderlich ist.

Davon ausgehend liegt der Erfindung die **Aufgabe** zugrunde, eine Vorrichtung der eingangs genannten Art zum Komprimieren rohrförmiger Endoprothesen sowie zum Einführen einer komprimierten Endoprothese in ein Applikationsrohr bereit zu stellen, die einfach zu handhaben ist und weitestgehend ohne manuellen Kontakt zur Endoprothese bedienbar ist.

Zur **Lösung** dieser Aufgabenstellung weist eine Vorrichtung gemäß dem Oberbegriff des Anspruchs 1 erfindungsgemäß eine aus mindestens zwei relativ zueinander bewegbaren Teilen bestehende rohrförmige Prothesenaufnahme auf, deren inneres Lumen im geschlossenen Zustand im Wesentlichen dem Außendurchmesser der komprimierten Endoprothese entspricht, sowie durch ein mitsamt der Endoprothese in die Prothesenaufnahme einsetzbares Faltwerkzeug zur Ausbildung einer nach innen gerichteten Längseinfaltung in der Endoprothese.

Durch den erfindungsgemäßen Aufbau der aus der Prothesenaufnahme und dem Faltwerkzeug bestehenden Vorrichtung ist es erstmalig möglich, eine Endoprothese weitestgehend ohne manuellen Kontakt zu komprimieren und in ein Applikationsrohr einzuführen. Darüber hinaus ermöglicht der einfache Aufbau der erfindungsgemäßen Vorrichtung eine einfache, schnelle und sichere Bedienung, bei der nicht die Gefahr besteht, dass die Endoprothese beschädigt wird.

Gemäß einer bevorzugten Ausführungsform der Erfindung besteht die Prothesenaufnahme aus zwei über ein Scharnier gegeneinander verschwenkbaren halbrohrförmigen Teilen, in die die Endoprothese eingelegt wird. Daraufhin lässt sich über das Scharnier, das beispielsweise als Filmscharnier ausgebildet sein kann, die Prothesenaufnahme dann zum Komprimieren der Endoprothese einfach verschließen, nachdem mittels des Faltwerkzeugs die nach innen gerichteten Längseinfaltung in der Endoprothese ausgebildet wurde.

Zum Festlegen aneinander sind die gegeneinander bewegbaren Teile der Prothesenaufnahme über einen Verriegelungsmechanismus, wie beispielsweise einen Schieberiegel, im geschlossenen Zustand der Prothesenaufnahme aneinander fixierbar.

Das Faltwerkzeug ist vorteilhafterweise als im wesentlichen zylindrischer Stab ausgebildet, da ein solcher zylindrischer Stab einfach und kostengünstig herstellbar ist und aufgrund seiner geometrischen Ausgestaltung nicht die Gefahr birgt, die Endoprothese während der Ausbildung der Einfaltung zu beschädigen.

Um das Einführen der komprimierten Endoprothese in das Applikationsrohr zu erleichtern, wird erfindungsgemäß vorgeschlagen, dass in der Prothesenaufnahme distalseitig ein das innere Lumen erweiternder Abschnitt zur Aufnahme des Applikationsrohres ausgebildet ist, wobei der Innendurchmesser des erweiterten Abschnitts im Wesentlichen dem Außendurchmesser des Applikationsrohres entspricht. Durch die Ausbildung dieses Abschnitts mit erweitertem Innendurchmesser wird in der Prothesenaufnahme eine Führungsaufnahme für das Applikationsrohr geschaffen.

Der Übergang von dem Abschnitt mit dem erweiterten Innendurchmesser zum kleineren Lumen der Prothesenaufnahme bildet dabei eine im wesentlichen kreisringförmige Anschlagfläche für das einzusetzende Applikationsrohr, wobei die radiale Höhe der Anschlagfläche mindestens der Wandstärke des Applikationsrohres entspricht, um eine Beschädigung der Endoprothese beim Einführen in das Applikationsrohr zu vermeiden.

Weiterhin wird gemäß einer praktischen Ausführungsform der Erfindung vorgeschlagen, dass die Vorrichtung eine Grundplatte zur Aufnahme der Prothesenaufnahme und des Faltwerkzeugs umfasst. Die Grundplatte besteht dabei vorzugsweise aus einer rechteckigen Basisplatte sowie zwei an einander gegenüberliegenden Seiten der Basisplatte angeordneten Stegen, wobei der Abstand zwischen den Stegen mindestens der axialen Länge der Prothesenaufnahme entspricht.

Zur sicheren und lagegenauen Lagerung der Prothesenaufnahme auf der Basisplatte der Grundplatte ist in der Basisplatte eine Führungsnut zur Aufnahme einer entsprechenden Führungsleiste der Prothesenaufnahme ausgebildet. Diese Führungsleiste der Prothesenaufnahme kann beispielsweise durch das die einzelnen Teile der Prothesenaufnahme miteinander verbindende Scharnier gebildet werden.

Das Festlegen des Faltwerkzeugs an der Grundplatte erfolgt erfindungsgemäß über in den Stegen ausgebildete Aussparungen zur Lagerung und Aufnahme des Faltwerkzeugs. Gemäß praktischer Ausgestaltungsformen wird vorgeschlagen, dass das distalseitige Ende des Faltwerkzeugs zur kippbeweglichen Lagerung in einem der Stege kugelförmig ausgebildet ist und im Bereich des proximalen Endes des Faltwerkzeugs am Faltwerkzeug ein Verriegelungselement angeordnet ist, über das das Faltwerkzeug in der entsprechend ausgebildeten Aussparung des anderen Stegs festlegbar ist.

Um die Handhabung des Faltwerkzeugs zu erleichtern, wird weiterhin vorgeschlagen, dass am proximalen Ende des Faltwerkzeugs ein Handgriff angeordnet ist.

Das Einführen der in der geschlossenen Prothesenaufnahme der erfindungsgemäßen Vorrichtung angeordneten komprimierten Endoprothese erfolgt über ein Ausdrückwerkzeug, mit dem die komprimierte Endoprothese aus der Prothesenaufnahme heraus in das Applikationsrohr verschiebbar ist.

Schließlich ist die mittels der erfindungsgemäßen Vorrichtung komprimierte Endoprothese über das Applikationsrohr endoskopisch oder radiologisch in ein Hohlorgan einsetzbar.

Weitere Merkmale und Vorteile der Erfindung ergeben sich anhand der nachfolgenden Beschreibung der zugehörigen Zeichnung, in der ein Ausführungsbeispiel einer erfindungsgemäßen Vorrichtung zum Komprimieren rohrförmiger Endoprothesen sowie zum Einführen einer komprimierten Endoprothese in ein Applikationsrohr nur beispielhaft schematisch dargestellt ist. In der Zeichnung zeigt:
- Fig. 1: eine perspektivische Explosionszeichnung einer erfindungsgemäßen-Vorrichtung in geöffneter Stellung mit eingelegter Endoprothese;
- Fig. 2: eine perspektivische Ansicht der Vorrichtung gemäß Fig. 1, jedoch die-Vorrichtung zusammengesetzt und in geschlossener Stellung darstellend;
- Fig. 3: eine perspektivische Ansicht der Prothesenaufnahme vor dem Ausschieben der komprimierten Endoprothese und
- Fig. 4: eine perspektivische Ansicht einer komprimierten Endoprothese.

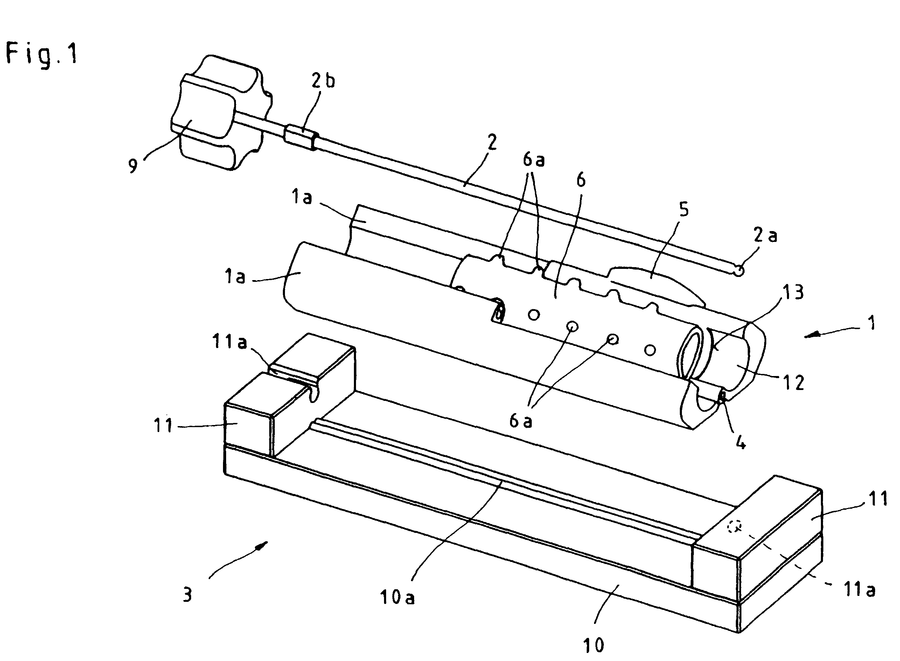

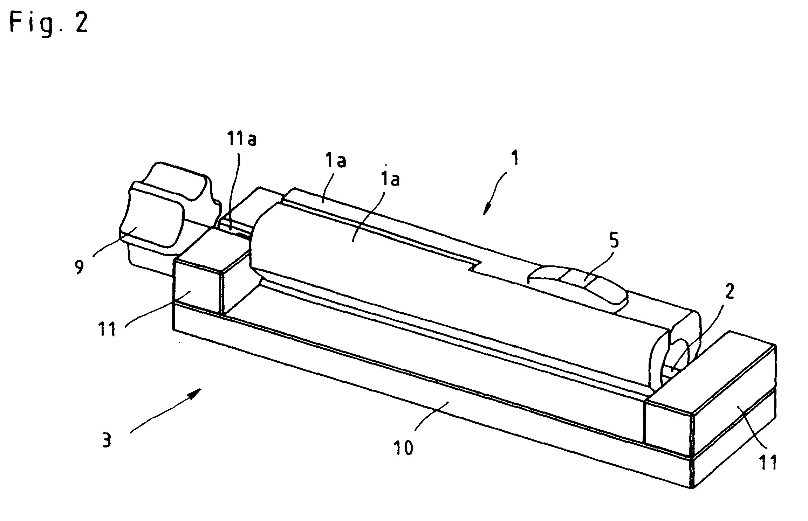

Die in den Abbildungen Fig. 1 und 2 dargestellte Vorrichtung zum Komprimieren rohrförmiger Endoprothesen sowie zum Einführen einer komprimierten Endoprothese in ein Applikationsrohr besteht im wesentlichen aus einer Prothesenaufnahme 1, einem Faltwerkzeug 2 sowie einer Grundplatte 3 zur Aufnahme und Lagerung der Prothesenaufnahme 1 sowie des Faltwerkzeugs 2.

Wie aus den Abbildungen weiterhin ersichtlich, besteht die Prothesenaufnahme 1 bei dem dargestellten Ausführungsbeispiel aus zwei halbrohrförmigen Teilen 1a, die über ein Scharnier 4 so miteinander verbundenen, dass die Prothesenaufnahme 1 über das Scharnier 4 zwischen einer geöffneten Stellung (Fig. 1) und einer geschlossenen Stellung (Fig. 2) verstellbar ist. Zum Fixieren der Prothesenaufnahme 1 in der geschlossenen Stellung weist die Prothesenaufnahme 1 einen Verriegelungsmechanismus 5 auf, über den die beiden Teile 1a an einander festlegbar sind.

Bei der dargestellten Ausführungsform ist der Verriegelungsmechanismus 5 als Schieberiegel ausgebildet. Selbstverständlich sind auch andere Ausgestaltungsformen des Verriegelungsmechanismus 5, wie beispielsweise Rast und Klemmhebel, einsetzbar. Ebenso ist der Aufbau der Prothesenaufnahme 1 nicht auf zwei gegeneinander verschwenkbare Teile 1a beschränkt, vielmehr ist es auch möglich, die Prothesenaufnahme 1 aus drei oder mehr Teilen 1a zu bilden, die durch Verschwenken, Verrasten, Stecken oder Ineinanderschieben zu einer geschlossenen rohrförmigen Prothesenaufnahme 1 zusammenfügbar sind.

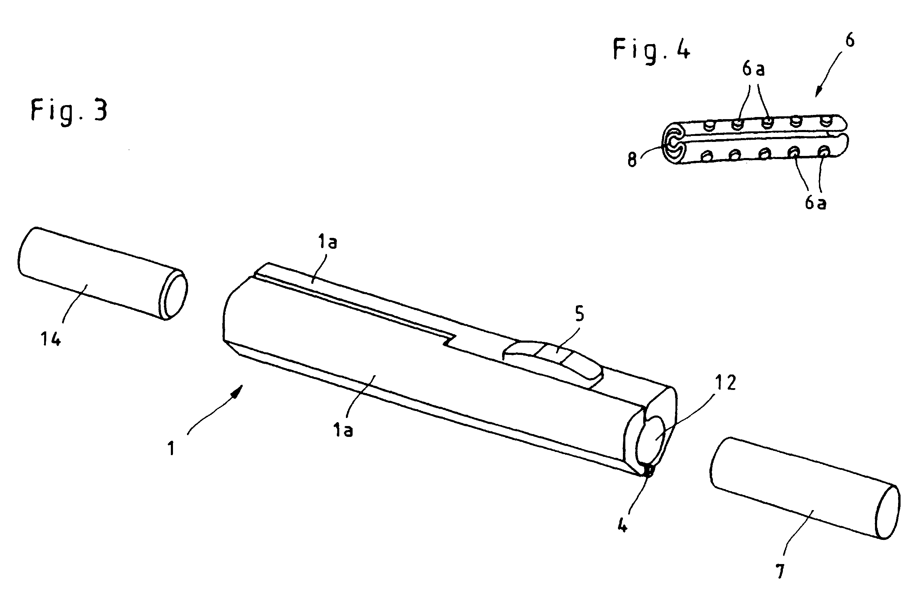

Wie in Fig. 1 dargestellt, dient die Prothesenaufnahme 1 zur Aufnahme einer rohrförmigen Endoprothese 6, die mittels der Prothesenaufnahme 1 und des Faltwerkzeugs 2 in den in Fig. 4 dargestellten komprimierten Zustand überführbar ist. In diesem komprimierten Zustand wird die Endoprothese 6 in ein Applikationsrohr 7 eingeführt und anschließend endoskopisch oder radiologisch in ein Hohlorgan, beispielsweise in die Luftröhre, in die Bronchien oder in Blutgefäße, eingesetzt, wo sich die Endoprothese 6 wieder selbsttätig entfaltet und die in Fig. 1 dargestellte Form einnimmt.

Bei der in Fig. 1 dargestellten Endoprothese 6 handelt es sich um eine einfache rohrförmige Endoprothese 6 auf deren äußeren Mantelfläche mehrere radial abstehende Noppen 6a angeordnet sind, die dazu dienen, dass sich die Endoprothese 6 gegen die Innenwand des betreffenden Hohlorgans abstützt und die Endoprothese lagegenau verankert. Neben der dargestellten Form einer einfachen rohrförmigen Endoprothese 6 ist es selbstverständlich auch möglich, anders geformte Endoprothesen 6, wie beispielsweise Y-Prothesen, mit dieser Vorrichtung zu komprimieren und in ein Applikationsrohr 7 einzuführen.

Da das Lumen der geschlossenen Prothesenaufnahme 1 deutlich geringer ist als der Außendurchmesser der entfalteten Endoprothese 6, wird beim Komprimieren der Endoprothese 6 in der Prothesenaufnahme 1 mittels des Faltwerkzeugs 2 eine Längseinfaltung 8 in der Endoprothese 6 ausgebildet, wie diese in Fig. 4 zu erkennen ist. Durch die Ausbildung dieser Längseinfaltung 8 verringert sich der Außendurchmesser der Endoprothese 6 deutlich und können die Seiten der eingefalteten Endoprothese 6 beim Schließen der Prothesenaufnahme 1 aufeinander zugedrückt werden, um so den Durchmesser der Endoprothese 6 weiter zu verringern.

Wie aus Fig. 1 ersichtlich, ist das Faltwerkzeug 2 zur Erzeugung der Längseinfaltung 8 in der Endoprothese 6 bei der dargestellten Ausführungsform als zylindrischer Stab ausgebildet, an dessen proximalem Ende ein Handgriff 9 angeordnet ist.

Die zur Aufnahme und Lagerung der Prothesenaufnahme 1 sowie des Faltwerkzeugs 2 dienende Grundplatte 3 besteht aus einer rechteckigen Basisplatte 10 sowie zwei an einander gegenüberliegenden Seiten der Basisplatte 10 angeordneten Stegen 11, wobei der Abstand zwischen den beiden Stegen 11 mindestens der axialen Länge der Prothesenaufnahme 1 entspricht. Zur lagegenauen Positionierung der Prothesenaufnahme 1 auf der Basisplatte 10 weist die Basisplatte 10 eine Führungsnut 10a auf, in die eine entsprechend geformte Führungsleiste der Prothesenaufnahme 1 einsetzbar ist. Bei der dargestellten Ausführungsform wird diese Führungsleiste durch das Scharnier 4 gebildet, über das die Teile 1a der Prothesenaufnahme 1 verschwenkbar miteinander verbunden sind.

Zur Lagerung des Faltwerkzeugs 2, das im vorliegenden Fall als zylindrischer Stab ausgebildet ist, sind in den Stegen 11 Aussparungen 11a ausgebildet, in die das Faltwerkzeug 2 einerseits über das kugelförmig ausgebildete distale Ende 2a und andererseits über ein Verriegelungselement 2b festlegbar ist, das im Bereich des proximalen Endes des Faltwerkzeugs 2 am Faltwerkzeug 2 ausgebildet ist.

Eine solchermaßen ausgestaltete Vorrichtung zum Komprimieren rohrförmiger Endoprothesen 6 sowie zum Einführen einer komprimierten Endoprothese 6 in ein Applikationsrohr 7 arbeitet wie folgt:

Um eine Endoprothese 6 mittels eines Applikationsrohres 7 in ein Hohlorgan implantieren zu können, ist es zunächst notwendig, die Endoprothese 6 aus der in Fig. 1 dargestellten vollständig entfalteten Form in die in Fig. 4 dargestellte Form zu überführen, in der die Endoprothese 6 zur späteren Implantation in ein Applikationsrohr 7 einsetzbar ist.

Im ersten Arbeitsschritt wird die vollständig entfaltete Endoprothese 6, wie aus Fig. 1 ersichtlich, in der offenen Stellung der Prothesenaufnahme 1 in die auf der Basisplatte 10 der Grundplatte 3 angeordnete Prothesenaufnahme 1 eingelegt.

Anschließend wird das Faltwerkzeug 2 mit seinem kugelförmigen distalen Ende 2a in die Aussparung 11a des in Fig. 1 und 2 rechten Stegs 11 eingesetzt und das Faltwerkzeug 2 herabbewegt, bis das im Bereich des proximalen Endes des Faltwerkzeugs 2 am Faltwerkzeug 2 angeordnete Verriegelungselement 2b Aufnahme in der Aussparung 11a des in Fig. 1 und 2 linken Stegs 11 findet. Bei dieser Abwärtsbewegung drückt das Faltwerkzeug 2 so auf die in der Prothesenaufnahme 1 gelagerte Endoprothese 6, dass sich in der Endoprothese 6 die in Fig. 4 dargestellte Längseinfaltung 8 bildet.

Das im Bereich des proximalen Endes des Faltwerkzeugs 2 angeordnete Verriegelungselement 2b dient dazu, das Faltwerkzeug 2 in der herabgedrückten Stellung an der Grundplatte 3 zu fixieren, damit das Faltwerkzeug 2 nicht durch die Materialelastizität der eingefalteten Endoprothese 6 wieder angehoben wird.

Im nachfolgenden Arbeitsschritt werden die halbrohrförmigen Teile 1a der Prothesenaufnahme 1 über das Scharnier 4 aufeinander zu verschwenkt, bis die Prothesenaufnahme 1 die in Fig. 2 dargestellte geschlossene Stellung einnimmt. In dieser Stellung sind die Teile 1a der Prothesenaufnahme 1 über den Verriegelungsmechanismus 5 aneinander fixierbar, so dass die Prothesenaufnahme 1 wieder von der Grundplatte 3 abgenommen werden kann, wie dies in Fig. 3 dargestellt ist.

In dieser von der Grundplatte 3 getrennten Stellung ist es nunmehr möglich, das Faltwerkzeug 2 mittels des Handgriffs 9 über die proximale Seite der Prothesenaufnahme 1 wieder aus der Prothesenaufnahme 1 heraus zu ziehen, in der sich jetzt nur noch die komprimierte Endoprothese 6 befindet.

Um die komprimierte Endoprothese 6 in das Applikationsrohr 7 überführen zu können, wird das Applikationsrohr 7', wie aus Fig. 3 ersichtlich, in das distale Ende der Prothesenaufnahme 1 eingeschoben. Zur sicheren Lagerung und Aufnahme des Applikationsrohres 7 ist in der Prothesenaufnahme 1 distalseitig ein das innere Lumen der Prothesenaufnahme 1 erweiternder Abschnitt 12 ausgebildet, wobei der Innendurchmesser des erweiterten Abschnitts 12 im Wesentlichen dem Außendurchmesser des Applikationsrohres 7 entspricht.

Das Applikationsrohr 7 wird dabei so weit in die Prothesenaufnahme 1 eingeschoben, bis das Apptikationsrohr 7 gegen die durch den Übergang von dem Abschnitt 12 mit dem erweiterten Innendurchmesser zum kleineren Lumen der Prothesenaufnahme 1 ausgebildete, im Wesentlichen kreisringförmige Anschlagfläche 13 anläuft.

Das eigentliche Überführen der komprimierten Endoprothese 6 in das Applikationsrohr 7 erfolgt mittels eines zylindrischen Ausdrückwerkzeugs 14, das über das proximale Ende in die Prothesenaufnahme 1 eingeführt wird. Durch Aufbringen weiterer Druckkraft auf das Ausdrückwerkzeug 14 wird die komprimierten Endoprothese 6 aus der Prothesenaufnahme 1 heraus in das Applikationsrohr 7 verschoben.

Damit die Endoprothese 6 beim Verschieben in das Applikationsrohr 7 nicht beschädigt wird, entspricht die radiale Höhe der Anschlagfläche 13 mindestens der Wandstärke des Applikationsrohres 7, so dass beim Übergang vom inneren Lumen der Prothesenaufnahme 1 hinein in das Applikationsrohr 7 nur eine unschädliche Durchmessererweiterung, niemals aber ein verengender und für die Endoprothese 6 gefährlicher Katibersprung auftreten kann.

Die nunmehr im Applikationsrohr 7 angeordnete komprimierte Endoprothese 6 kann nachfolgend endoskopisch oder radiologisch in ein Hohlorgan eingesetzt werden.

Eine solchermaßen ausgestaltete Vorrichtung zum Komprimieren rohrförmiger Endoprothesen 6 sowie zum Einführen einer komprimierten Endoprothese 6 in ein Applikationsrohr 7 zeichnet sich somit dadurch aus, dass die Vorrichtung weitestgehend ohne manuellen Kontakt zur Endoprothese 6 bedienbar ist und aufgrund des einfachen Aufbaus einfach, sicher und schnell zu handhaben ist.

### Bezugszeichenliste

- 1: Prothesenaufnahme
- 1a: Teil
- 2: Faltwerkzeug
- 2a: distales Ende
- 2b: Verriegelungselement
- 3: Grundplatte
- 4: Scharnier
- 5: Verriegelungsmechanismus
- 6: Endoprothese
- 6a: Noppen
- 7: Applikationsrohr
- 8: Längseinfaltung
- 9: Handgriff
- 10: Basisplatte
- 10a: Führungsnut
- 11: Steg
- 11a: Aussparung
- 12: erweiterter Abschnitt
- 13: Anschlagfläche
- 14: Ausdrückwerkzeug

## Patentansprüche

1. Vorrichtung zum Komprimieren rohrförmiger Endoprothesen sowie zum Einführen einer komprimierten Endoprothese in ein Applikationsrohr,
**gekennzeichnet durch**
eine aus mindestens zwei relativ zueinander bewegbaren Teilen (1a) bestehende rohrförmige Prothesenaufnahme (1), deren inneres Lumen im geschlossenen Zustand im Wesentlichen dem Außendurchmesser der komprimierten Endoprothese (6) entspricht, sowie **durch** ein mitsamt der Endoprothese (6) in die Prothesenaufnahme (1) einsetzbares Faltwerkzeug (2) zur Ausbildung einer nach innen gerichteten Längseinfaltung (8) in der Endoprothese (6).

2. Vorrichtung nach Anspruch 1, **dadurch gekennzeichnet, dass** die Prothesenaufnahme (1) aus zwei über ein Scharnier (4) gegeneinander verschwenkbaren halbrohrförmigen Teilen (1a) besteht.

3. Vorrichtung nach Anspruch 2, **dadurch gekennzeichnet, dass** das Scharnier (4) als Filmscharnier ausgebildet ist.

4. Vorrichtung nach mindestens einem der Ansprüche 1 bis 3, **dadurch gekennzeichnet, dass** die gegeneinander bewegbaren Teile (1a) der Prothesenaufnahme (1) über einen Verriegelungsmechanismus (5) im geschlossenen Zustand der Prothesenaufnahme (1) aneinander fixierbar sind.

5. Vorrichtung nach mindestens einem der Ansprüche 1 bis 4, **dadurch gekennzeichnet, dass** das Faltwerkzeug (2) als im wesentlichen zylindrischer Stab ausgebildet ist.

6. Vorrichtung nach mindestens einem der Ansprüche 1 bis 5, **dadurch gekennzeichnet, dass** die Vorrichtung ein Applikationsrohr (7) umfasst, und dass in der Prothesenaufnahme (1) distalseitig ein das innere Lumen erweiternder Abschnitt (12) zur Aufnahme des Applikationsrohres (7) ausgebildet ist, wobei der Innendurchmesser des erweiterten Abschnitts (12) im Wesentlichen dem Außendurchmesser des Applikationsrohres (7) entspricht.

7. Vorrichtung nach Anspruch 6, **dadurch gekennzeichnet, dass** der Übergang von dem Abschnitt (12) mit dem erweiterten Innendurchmesser zum kleineren Lumen der Prothesenaufnahme (1) eine im wesentlichen kreisringförmige Anschlagfläche (13) für das einzusetzende Apptikationsrohr (7) bildet, wobei die radiale Höhe der Anschlagfläche (13) mindestens der Wandstärke des Applikationsrohres (7) entspricht.

8. Vorrichtung nach mindestens einem der Ansprüche 1 bis 7, **gekennzeichnet durch** eine Grundplatte (3) zur Aufnahme der Prothesenaufnahme (1) sowie des Faltwerkzeugs (2).

9. Vorrichtung nach Anspruch 8, **dadurch gekennzeichnet, dass** die Grundplatte (3) aus einer rechteckigen Basisplatte (10) sowie zwei an einander gegenüberliegenden Seiten der Basisplatte (10) angeordneten Stegen (11) besteht, wobei der Abstand zwischen den Stegen (11) mindestens der axialen Länge der Prothesenaufnahme (1) entspricht.

10. Vorrichtung nach Anspruch 9, **dadurch gekennzeichnet, dass** in der Basisplatte (10) eine Führungsnut (10a) zur Aufnahme einer entsprechenden Führungsleiste der Prothesenaufnahme (1) ausgebildet ist.

11. Vorrichtung nach Anspruch 10, **dadurch gekennzeichnet, dass** das Scharnier (4) der Prothesenaufnahme (1) die Führungsleiste bildet.

12. Vorrichtung nach mindestens einem der Ansprüche 9 bis 11, **dadurch gekennzeichnet, dass** in den Stegen (11) Aussparungen (11a) zur Lagerung und Aufnahme des Faltwerkzeugs (2) ausgebildet sind.

13. Vorrichtung nach Anspruch 12, **dadurch gekennzeichnet, dass** das distalseitige Ende (2a) des Faltwerkzeugs (2) zur kippbeweglichen Lagerung in einem der Stege (11) kugelförmig ausgebildet ist.

14. Vorrichtung nach Anspruch 12 oder 13, **dadurch gekennzeichnet, dass** im Bereich des proximalen Endes des Faltwerkzeugs (2) am Faltwerkzeug (2) ein Verriegelungselement (2b) angeordnet ist, über das das Faltwerkzeug (2) in der entsprechend ausgebildeten Aussparung (11a) in einem der Stege (11) festlegbar ist.

15. Vorrichtung nach mindestens einem der Ansprüche 1 bis 14, **dadurch gekennzeichnet, dass** am proximalen Ende des Faltwerkzeugs (2) ein Handgriff (9) angeordnet ist.

16. Vorrichtung nach mindestens einem der Ansprüche 1 bis 15, **dadurch gekennzeichnet, dass** die in der geschlossenen Prothesenaufnahme (1) angeordnete komprimierte Endoprothese (6) über ein Ausdrückwerkzeug (14) aus der Prothesenaufnahme (1) heraus in das Applikationsrohr (7) verschiebbar ist.

## Claims

1. Device for compressing tubular endoprostheses and for inserting a compressed endoprosthesis into an application tube, **characterized by** a tubular prosthesis holder (1) which is made up of at least two parts (1a) that can move relative to one another and whose inner lumen, in the closed state, corresponds substantially to the external diameter of the compressed endoprosthesis (6), and by a folding tool (2) which can be introduced together with the endoprosthesis (6) into the prosthesis holder (1) and which is used to form an inwardly directed lengthwise fold (8) in the endoprosthesis (6).

2. Device according to Claim 1, **characterized in that** the prosthesis holder (1) is made up of two semitubular parts (1a) which can pivot relative to one another via a hinge (4).

3. Device according to Claim 2, **characterized in that** the hinge (4) is configured as a film hinge.

4. Device according to at least one of Claims 1 to 3, **characterized in that** the parts (1a) of the prosthesis holder (1) which are movable relative to one another can be secured on one another by means of a locking mechanism (5) in the closed state of the prosthesis holder (1).

5. Device according to at least one of Claims 1 to 4, **characterized in that** the folding tool (2) is configured as a substantially cylindrical rod.

6. Device according to at least one of Claims 1 to 5, **characterized in that** the device comprises an application tube (7), and **in that**, in the prosthesis holder (1), at its distal end, a section (12) that widens the inner lumen is formed for receiving the application tube (7), the internal diameter of the widened section (12) corresponding substantially to the external diameter of the application tube (7).

7. Device according to Claim 6, **characterized in that** the transition from the section (12) with the widened internal diameter to the smaller lumen of the prosthesis holder (1) forms a substantially circular abutment surface (13) for the application tube (7) to be introduced, the radial height of the abutment surface (13) corresponding to at least the wall thickness of the application tube (7).

8. Device according to at least one of Claims 1 to 7, **characterized by** a plate (3) for receiving the prosthesis holder (1) and the folding tool (2).

9. Device according to Claim 8, **characterized in that** the plate (3) is made up of a rectangular base plate (10) and of two bridges (11) arranged at opposite ends of the base plate (10), the distance between the bridges (11) corresponding at least to the axial length of the prosthesis holder (1).

10. Device according to Claim 9, **characterized in that** a guide groove (10a) is formed in the base plate (10) for the purpose of receiving a corresponding guide ridge of the prosthesis holder (1).

11. Device according to Claim 10, **characterized in that** the hinge (4) of the prosthesis holder (1) forms the guide ridge.

12. Device according to at least one of Claims 9 to 11, **characterized in that** recesses (11a) are formed in the bridges (11) for mounting and supporting of the folding tool (2).

13. Device according to Claim 12, **characterized in that** the distal end (2a) of the folding tool (2) has a spherical shape in order to permit tiltable mounting in one of the bridges (11).

14. Device according to Claim 12 or 13, **characterized in that**, in the area of the proximal end of the folding tool (2), a locking element (2b) is formed on the folding tool (2) and can secure the folding tool (2) in the correspondingly configured recess (11a) in one of the bridges (11).

15. Device according to at least one of Claims 1 to 14, **characterized in that** a handle (9) is arranged at the proximal end of the folding tool (2).

16. Device according to at least one of Claims 1 to 15, **characterized in that** the compressed endoprosthesis (6) arranged in the closed prosthesis holder (1) can be slid out of the prosthesis holder (1) and into the application tube (7) by means of an ejection tool (14).

## Revendications

1. Dispositif pour comprimer des endoprothèses de forme tubulaires, ainsi que pour introduire une endoprothèse comprimée dans un tube d'application, **caractérisé par** un logement de réception de prothèse (1) constitué d'au moins deux parties (1a) mobiles relativement l'une par rapport à l'autre, dont la lumière intérieure, à l'état fermé, correspond sensiblement au diamètre extérieur de l'endoprothèse (6) comprimée, ainsi que par un outil de pliage (2) pouvant être introduit en commun avec l'endoprothèse (6) dans le logement de réception de prothèse (1) et destiné à former dans l'endoprothèse (6), un repli longitudinal (8) dirigé vers l'intérieur.

2. Dispositif selon la revendication 1, **caractérisé en ce que** le logement de réception de prothèse (1) est constitué de deux parties (1a) en forme de demi-tube pouvant basculer l'une vers l'autre par l'intermédiaire d'une charnière (4).

3. Dispositif selon la revendication 2, **caractérisé en ce que** la charnière (4) est réalisée sous forme de charnière à film.

4. Dispositif selon l'une au moins des revendications 1 à 3, **caractérisé en ce que** les parties (1a) mobiles l'une vers l'autre, du logement de réception de prothèse (1), peuvent être fixées l'une à l'autre à l'aide d'un mécanisme de verrouillage (5), dans l'état fermé du logement de réception de prothèse (1).

5. Dispositif selon l'une au moins des revendications 1 à 4, **caractérisé en ce que** l'outil de pliage (2) est réalisé sous la forme d'une tige essentiellement cylindrique.

6. Dispositif selon l'une au moins des revendications 1 à 5, **caractérisé en ce que** le dispositif comprend un tube d'application (7), et **en ce que** dans le logement de réception de prothèse (1) est formé, côté distal, un tronçon (12) qui élargit la lumière intérieure et est destiné à recevoir le tube d'application (7), le diamètre intérieur du tronçon élargi (12) correspondant sensiblement au diamètre extérieur du tube d'application (7).

7. Dispositif selon la revendication 6, **caractérisé en ce que** la transition du tronçon (12) de diamètre intérieur élargi à la lumière plus petite du logement de réception de prothèse (1), réalise une surface de butée (13) sensiblement de forme annulaire circulaire destinée au tube d'application (7) à insérer, la hauteur radiale de la surface de butée (13) correspondant au moins à l'épaisseur de paroi du tube d'application (7).

8. Dispositif selon l'une au moins des revendications 1 à 7, **caractérisé par** une plaque de support (3) destinée à recevoir le logement de réception de prothèse (1) ainsi que l'outil de pliage (2).

9. Dispositif selon la revendication 8, **caractérisé en ce que** la plaque de support (3) est constituée d'une plaque de base (10) rectangulaire ainsi que de deux nervures (11) disposées sur deux côtés opposés de la plaque de base (10), la distance entre les nervures (11) correspondant au moins à la longueur axiale du logement de réception de prothèse (1).

10. Dispositif selon la revendication 9, **caractérisé en ce que** dans la plaque de base (10) est formée une rainure de guidage (10a) destinée à recevoir une barrette de guidage correspondante du logement de réception de prothèse (1).

11. Dispositif selon la revendication 10, **caractérisé en ce que** la charnière (4) du logement de réception de prothèse (1) forme la barrette de guidage.

12. Dispositif selon l'une au moins des revendications 9 à 11, **caractérisé en ce que** dans les nervures (11) sont formés des évidements (11a) destinés au montage et à la réception de l'outil de pliage (2).

13. Dispositif selon la revendication 12, **caractérisé en ce que** l'extrémité (2a), côté distal, de l'outil de pliage (2) est réalisée en forme de rotule sphérique pour son montage à mobilité de basculement dans l'une des nervures (11).

14. Dispositif selon la revendication 12 ou 13, **caractérisé en ce que** dans la zone de l'extrémité proximale de l'outil de pliage (2) est disposé, sur l'outil de pliage (2), un élément de verrouillage (2b) par lequel l'outil de pliage (2) peut être fixé dans l'évidement (11a) de configuration correspondante dans l'une des nervures (11).

15. Dispositif selon l'une au moins des revendications 1 à 14, **caractérisé en ce qu'**à l'extrémité proximale de l'outil de pliage (2) est disposée une poignée (9).

16. Dispositif selon l'une au moins des revendications 1 à 15, **caractérisé en ce que** l'endoprothèse (6) comprimée, disposée dans le logement de réception de prothèse (1) fermé, peut être repoussée par coulissement hors du logement de réception de prothèse (1) dans le tube d'application (7), à l'aide d'un outil de repoussage (14).
